# FASCICULE DE BREVET EUROPEEN

(11) **EP 1 406 874 B1**
(45) Date de publication et mention de la délivrance du brevet: **28.03.2007**
(21) Numéro de dépôt: 02764952.4
(22) Date de dépôt: 09.07.2002
(51) Int. Cl.: C07D 231/38, A61Q 5/10

(54) **COMPOSES DERIVES DE DIAMINOPYRAZOLE SUBSTITUES PAR UN RADICAL AMINOALKYLE OU AMINOALCENYLE ET LEUR UTILISATION EN TEINTURE D'OXYDATION DES FIBRES KERATINIQUES**
DURCH AMINALKYL- ODER AMINOALKENYL-RESTE SUBSTITUIERTE DIAMINOPYRAZOLDERIVATE UND IHRE ANWENDUNG FÜR DIE OXYDATIVE FÄRBUNG VON KERATINFASERN
COMPOUNDS DERIVED FROM DIAMINOPYRAZOLES SUBSTITUTED BY AN AMINOALKYL OR AMINOALKENYL RADICAL AND THEIR USE IN OXIDATION DYEING OF KERATINOUS FIBRES

(30) Priorité: 18.07.2001 FR 0109623
(43) Date de publication de la demande: 14.04.2004
(73) Titulaire: L'ORÉAL, 75008 Paris (FR)
(72) Inventeur: FESSMANN, Thilo, F-93600 Aulnay-sous-Bois (FR); TERRANOVA, Eric, F-06520 Magagnosc (FR)
(74) Mandataire: Casalonga, Axel
(86) Numéro de dépôt international: PCT/FR2002/002396
(87) Numéro de publication internationale: WO 2003/008385

(56) Documents cités:
- EP-A- 0 740 931
- WO-A-00/43367
- WO-A-94/08969
- WO-A-98/17234
- WO-A-98/20847

## Description

La présente invention est relative à de nouveaux composés dérivés de diaminopyrazole, à une composition pour la teinture d'oxydation des fibres kératiniques, et en particulier des fibres kératiniques humaines telles que les cheveux, comprenant au moins, comme base d'oxydation, un composé dérivé de diaminopyrazole et aux procédés de teinture d'oxydation la mettant en oeuvre.

Il est connu de teindre les fibres kératiniques et en particulier les cheveux humains avec des compositions tinctoriales contenant des précurseurs de colorant d'oxydation, en particulier des ortho- ou paraphénylènediamines, des ortho- ou para-aminophénols, des composés hétérocycliques tels que des dérivés de diaminopyrazole, appelés généralement bases d'oxydation. Les précurseurs de colorants d'oxydation, ou bases d'oxydation, sont des composés, incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les méta-aminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire à un certain nombre d'exigences. Ainsi, elle doit être sans inconvénient sur le plan toxicologique, elle doit permettre d'obtenir des nuances dans l'intensité souhaitée, présenter une bonne tenue aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possibles tout au long d'une même fibre kératinique, qui peut être en effet différemment sensibilisée (c'est-à-dire abîmée) entre sa pointe et sa racine. Ils doivent également présenter une bonne stabilité chimique dans les formulations et doivent présenter un bon profil toxicologique.

De plus, pour un certain nombre d'applications, on recherche des colorants donnant sur les cheveux des nuances chromatiques.

On connaît par la demande EP 375 977 des composés 4,5-diaminopyrazoles utiles comme agent de coloration en teinture d'oxydation. On connaît également dans la demande de brevet EP 871 426 des compositions contenant des dérivés 4,5-diaminopyrazoles substitués en position 1 par un groupe alkyle, mono ou polyhydroxyalkyle ou mono ou polyaminoalkyle associés à des coupleurs particuliers aminophénol et m-phénylènediamine.

Cependant, ces colorants ne permettent pas de satisfaire à toutes les exigences ci-dessus.

Or, la demanderesse vient maintenant de découvrir, de façon totalement inattendue et surprenante, qu'il était possible d'obtenir des teintures, capables de conduire à des colorations puissantes, particulièrement chromatiques et brillantes, peu sélectives, et présentant d'excellentes propriétés de résistance aux diverses agressions que peuvent subir les fibres kératiniques en utilisant à titre de base d'oxydation les diaminopyrazoles de formule (I) ci-après ou leurs sels physiologiquement acceptables.

La présente invention a donc pour objet des composés dérivés de diaminopyrazoles ayant la structure suivante : 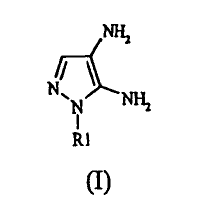 dans laquelle R₁ est un radical linéaire ou ramifié choisi parmi les radicaux aminoalkyles en C₂, C₃, C₄ ou aminoalcényles en C₂, C₃, C₄.

Par radical alkyle en C₂, C₃, C₄, on entend un radical hydrocarboné à 2, 3 ou 4 atomes de carbone ne comprenant pas d'insaturation, de manière particulière il s'agira des radicaux éthyle, n-propyle, iso-propyle, n-butyle; isobutyle, tertiobutyle, 2-méthylpropyle.

Par radical alcényle en C₂, C₃, C₄, on entend un radical hydrocarboné à 2, 3 ou 4 atomes de carbone et comprenant une ou deux doubles liaisons.

L'invention a également pour objet les sels d'acide physiologiquement acceptables des composés de formule (I) tels que les chlorhydrates, les bromhydrates, les sulfates, les tartrates, les lactates ou les acétates.

L'invention a également pour objet une composition pour la teinture d'oxydation des fibres kératiniques, et en particulier des fibres kératiniques humaines telles que les cheveux, caractérisée par le fait qu'elle contient, dans un milieu approprié pour la teinture, comme base d'oxydation au moins un diaminopyrazole de formule (I) ci-dessus, ou ses sels d'acides physiologiquement acceptables.

Comme indiqué précédemment, les colorations obtenues avec la composition de teinture d'oxydation conforme à l'invention sont puissantes, particulièrement brillantes et chromatiques. Elles permettent d'atteindre en particulier des nuances rouges exemptes ou contenant très peu de bleu ou de jaune. Elles présentent de plus d'excellentes propriétés de résistance vis-à-vis de l'action des différents agents extérieurs (lumière, intempéries, lavage, ondulation permanente, transpiration, frottements).

L'invention a aussi pour objet un procédé de teinture d'oxydation des fibres kératiniques mettant en oeuvre une telle composition tinctoriale.

A titre d'exemples de diaminopyrazoles de formule (I) selon l'invention, on peut citer les composés suivants :

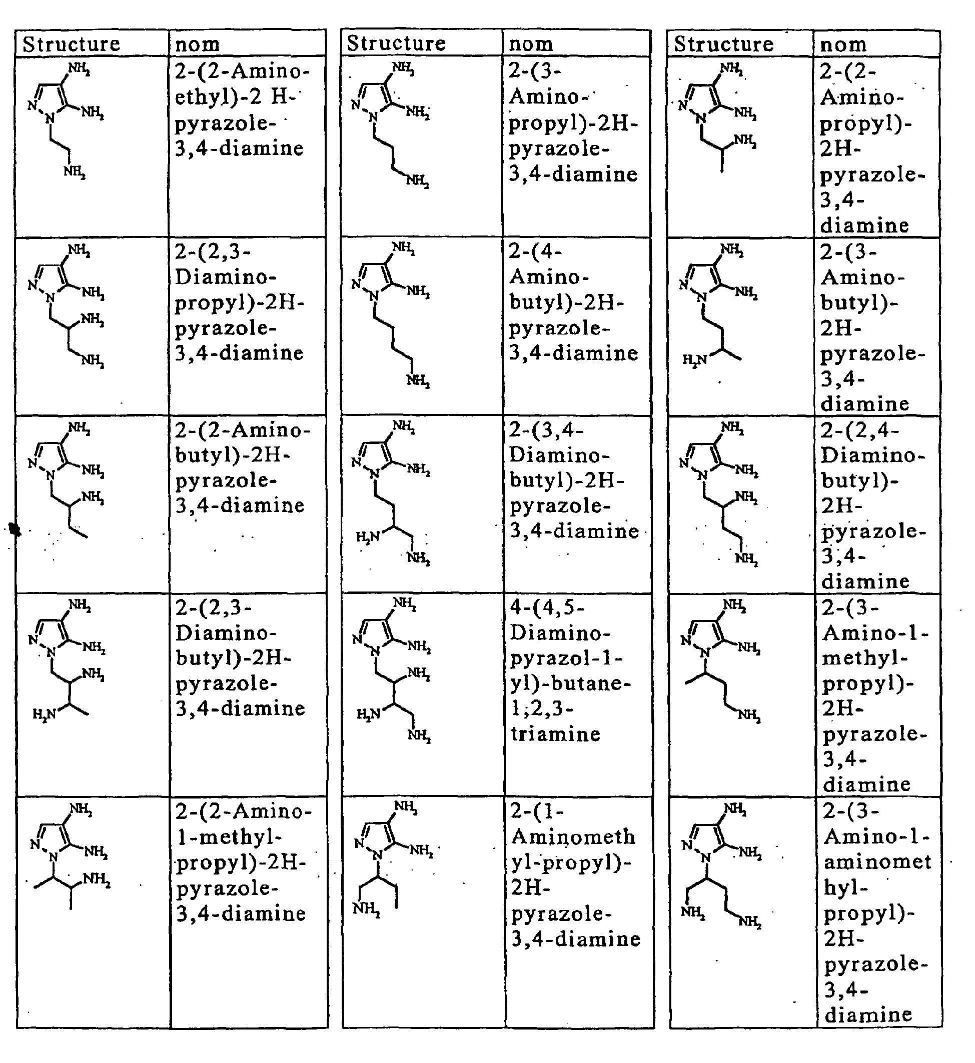

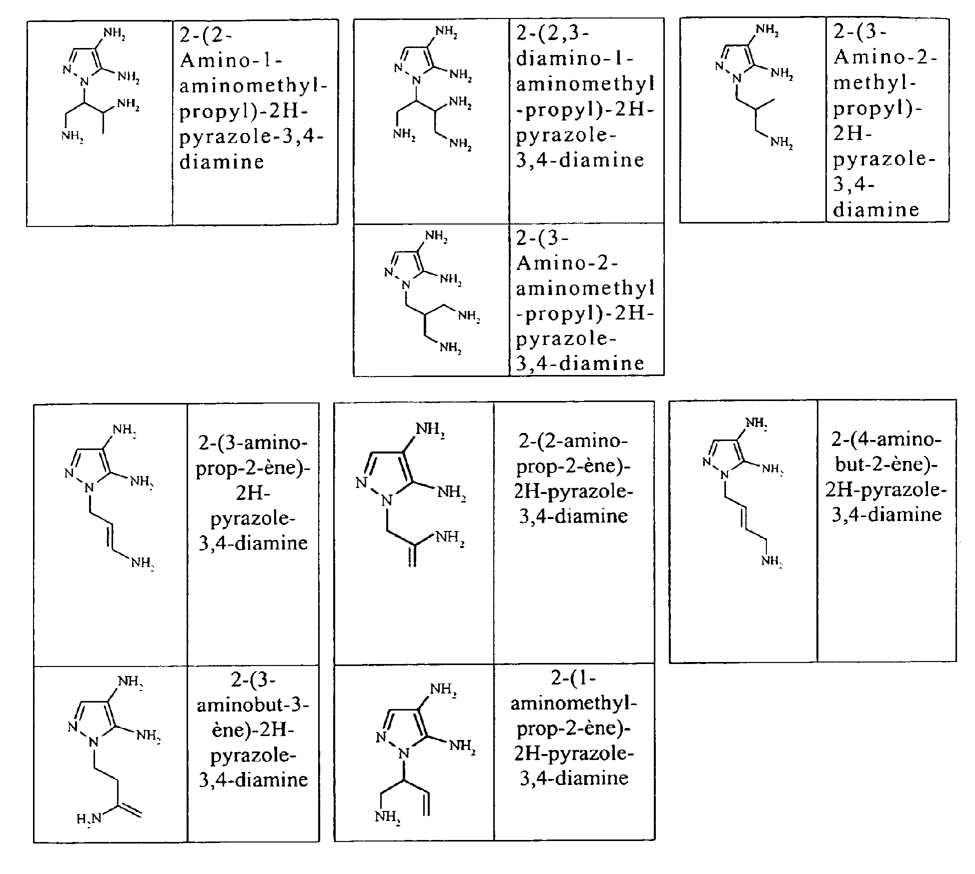

Les diaminopyrazoles de formule (I) préférés selon l'invention ont les structures suivantes :

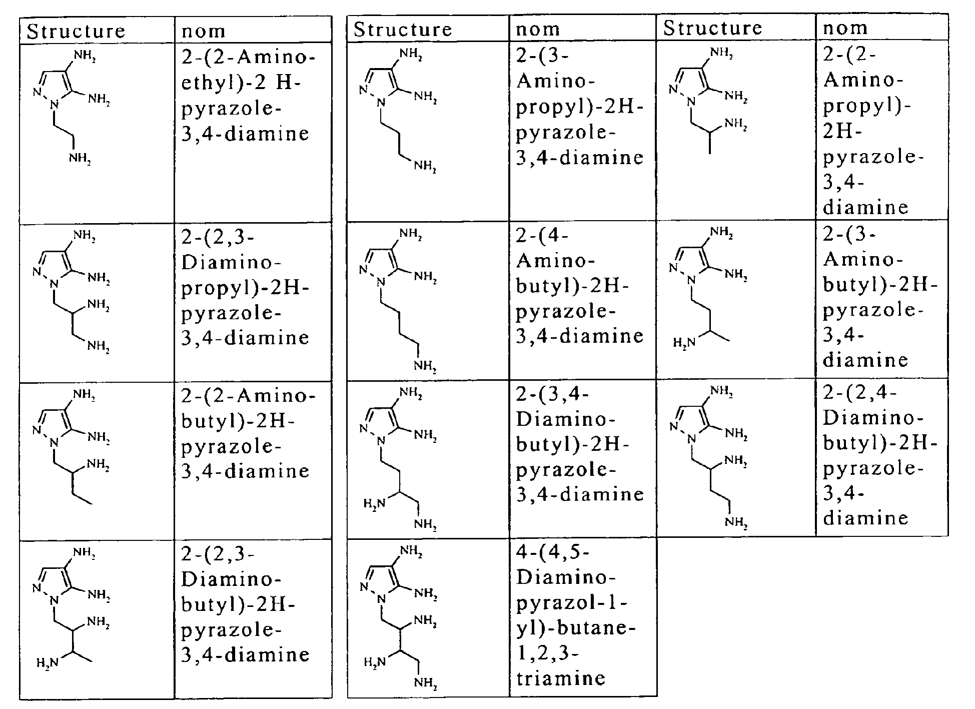

Les diaminopyrazoles de formule (I) plus particulièrement préférés selon l'invention sont 2-(2-aminoéthyl)-2 H-pyrazole 3,4-diamine, 2-(3-aminopropyle)-2H-pyrazole-3,4-diamine, 2-(2-aminopropyle)-2H-pyrazole-3,4-diamine, 2-(4-aminobutyle)-2H-pyrazole-3,4-diamine et 2-(3-aminobutyle)-2H-pyrazole-3,4-diamine ou leurs sels d'addition avec des acides physiologiquement acceptables.

Les diaminopyrazoles de formule (I) selon l'invention sont préparés par exemple selon la méthode générale de préparation suivante :

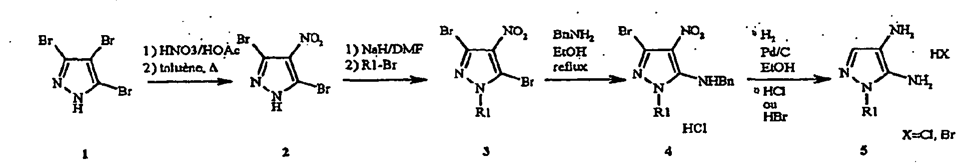

L'approche synthétique montrée ci-dessous est décrite dans la littérature jusqu'à l'intermédiaire (2) (J. H. P. Juffermanns, C. L ; Habraken ; J. Org. Chem., 1986, 51, 4656 ; Klebe et al. ; Synthesis, 1973, 294 ; R. Hüttel, F. Büchele ; Chem. Ber. ; 1955, 88, 1586.).

L'alkylation et l'amination pour arriver aux composés du type (5) de formule (I) selon l'invention sont mentionnées par exemple dans le document DE 42 34 885.

La composition tinctoriale selon l'invention contient notamment de 0,001 à 10% en poids, de préférence de 0,05 à 6% en poids, et encore plus préférentiellement de 0,1 à 3% en poids, d'au moins un diaminopyrazole de formule (I) ou ses sels.

La composition tinctoriale conforme à l'invention peut aussi contenir, en plus du (ou des) diaminopyrazole(s) défini(s) ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et des bases hétérocycliques différentes des diaminopyrazoles utilisés conformément à l'invention.

Parmi les paraphénylènediamines, on peut plus particulièrement citer à titre d'exemple, la paraphénylènediamine, la paratoluènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β**-**méthoxyéthyl) aniline, les paraphénylènediamines décrites dans la demande de brevet français FR 2630438, et leurs sels d'addition.

Parmi les bis-phénylalkylènediamines, on peut plus particulièrement citer à titre d'exemple, le N,N'-bis-(β-hydroxyéthyl) N,N'-bis-(4'-aminophényl) 1,3-diaminopropanol, la N,N'-bis-(β-hydroxyéthyl) N,N'-bis-(4'-aminophényl) éthylène-diamine, la N,N'-bis-(4-amino phényl) tétra-méthylènediamine, la N,N'-bis-(β**-**hydroxyéthyl), N,N'-bis-(4-aminophényl) tétraméthylènediamine, la N,N'-bis-(4-méthylaminophényl) tétraméthylène-diamine, la N,N'-bis-(éthyl) N,N'-bis-(4'-amino, 3'-méthylphényl) éthylènediamine, et leurs sels d'addition.

Parmi les para-aminophénols, on peut plus particulièrement citer à titre d'exemple, le para-aminophénol, le 4-amino 3-méthyl phénol, le 4-amino 3- fluoro phénol, le 4-amino 3-hydroxy-méthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β**-**hydroxyéthyl aminométhyl) phénol, et leurs sels d'addition.

Parmi les ortho-aminophénols, on peut plus particulièrement citer à titre d'exemple, le 2-amino phénol, le 2-amino 5-méthyl phénol, le 2-amino 6-méthylphénol, le 5-acétamido 2-amino phénol, et leurs sels d'addition.

Parmi les bases hétérocycliques, on peut plus particulièrement citer à titre d'exemple, les dérivés pyridiniques, les dérivés pyrimidiniques, les dérivés pyrazoliques différents des diamino pyrazoles de formule (I) utilisés conformément à l'invention, et leurs sels d'addition.

Lorsqu'elles sont utilisées, ces bases d'oxydation additionnelles représentent de préférence de 0,0005 à 12% en poids du poids total de la composition tinctoriale, et encore plus préférentiellement de 0,005 à 6% en poids de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols, les dérivés mono ou polyhydroxylé du naphtalène et les coupleurs hétérocycliques tels que par exemple les dérivés indoliques ou pyridiniques et leurs sels d'addition.

Ces coupleurs sont plus particulièrement choisis parmi le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy-N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Lorsqu'ils sont présents, ces coupleurs représentent notamment de 0,0001 à 10% du poids total de la composition tinctoriale, de préférence de 0,005 à 5% en poids, et encore plus préférentiellement de 0,1 à 3% de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates, les tartrates, les lactates et les acétates.

Le milieu approprié pour la teinture (ou support) utilisé selon l'invention est constitué par de l'eau ou par un mélange d'eau et d'au moins un solvant organique choisi parmi les alcanols inférieurs en C₁-C₄, les polyols et éthers de polyols, les alcools aromatiques, les produits analogues et leurs mélanges.

La composition tinctoriale selon l'invention peut également contenir divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, des agents antioxydants, des agents réducteurs, des filtres solaires, des agents de pénétration, des agents séquestrants, des parfums, des tampons, des agents dispersants, des agents de conditionnement tels que par exemple des silicones, des agents filmogènes, des agents conservateurs, des agents opacifiants.

Le pH de la composition tinctoriale selon l'invention est compris entre 3 et 12.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes, de préférence 5 à 30 minutes, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLES

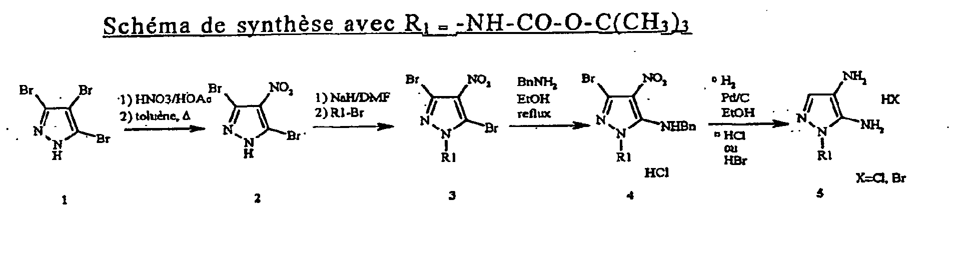

### Exemple de synthèse : synthèse du bromhydrate de la 2-(2-Amino-ethyl)-2H-pyrazole-3,4-diamine :

### Synthèse du 3,4,5-tribromopyrazole (1):

Une solution aqueuse (350 ml) contenant la soude (24 g, 0.6 moles) et le pyrazole (10 g, 0.147 moles) a été préparée sous agitation (la température du milieu réactionnel est montée jusqu'à 35°C). Après refroidissement du milieu réactionnel à 20°C, Br₂ (72 g, 0,45 mole) a été ajouté goutte à goutte pendant lh en maintenant la température entre 20°C et 25°C. La réaction a été suivie par chromatographie sur couche mince (CCM). Le précipité a été filtré et lavé avec de l'eau déminéralisée (100 ml). Le filtrat a été acidifié à pH 6-7 en utilisant HCl (10%, 33 g, 0,27 mole) et en maintenant la température entre 20 et 25°C. Le précipité ainsi formé est filtré et lavé avec de l'eau déminéralisée (100 ml). Les solides combinés sont portés à reflux dans un appareil de Dean-Stark en présence de toluène (200 ml). A la fin de la collection de l'eau, la phase organique est filtrée à chaud. Le solvant est évaporé jusqu'à un volume résiduel de 110 ml. La solution est refroidie à 0-5°C pendant 1h. Le précipité formé est collecté par filtration, lavé avec du toluène froid (20 ml) et séché sous vide à 80°C pour donner le 3,4,5-tribromopyrazole (1) sous forme de solide blanc cassé (30 g, 67%).
¹³C - RMN : (100 MHz, d6-DMSO) :97,7 - 116,1 - 126,4
Point de fusion. : 182-184°C

### Synthèse du 3,5-dibromo-4-nitropyrazole (2):

HNO₃ (d=1,50 g/ml; 18 ml, 0,429 mole) a été ajouté goutte à goutte pendant 10 min à une solution de 3,4,5-tribromopyrazole (1) (50 g, 0,164 mole) dans l'acide acétique glacial (750 ml) en maintenant la température à 15°C. De l'anhydride acétique (250 ml) à été ajoutée et le mélange réactionnel a été agité à température ambiante pendant 2 h. Après réaction complète, le mélange réactionnel a été versé sur de la glace brisée (1 kg). Après 1 h d'agitation, le produit brut a été filtré, puis lavé avec de l'eau déminéralisée (2 x 60 ml) pour donner du 1-nitro-3,4,5-tribromopyrazole brut. L'eau (24,6 ml) contenue dans le produit humide a été enlevée en chauffant une solution du produit dans du toluène (750 ml) à reflux dans un appareil de Dean-Stark. La solution toluénique a été maintenue à reflux pendant 30 min supplémentaires jusqu'à ce qu'une CCM (éluant toluène) indique que le réarrangement du 1-nitro-3,4,5-tribromopyrazole (Rf = 0,77), intermédiairement formé, en 3,5-dibromo-4-nitropyrazole 2 (Rf = 0,05) est complète. La solution a été concentrée jusqu'à un volume résiduel de 150 ml, puis laissée refroidir jusqu'à 60°C avant d'ajouter de l'hexane (275 ml). La solution a été refroidie à 0-5°C pendant lh et le 3,5-dibromo-4-nitropyrazole (2) (29,1 g, 65%) a été récupéré par filtration et séchage sous vide sous forme de solide jaune-clair.
Point de fusion . : 127,6-130,1°C.

### Synthèse de [2-(3,5-Dibromo-4-nitro-pyrazol-1-yl)-ethyl]-carbamic acid tert-butyl ester (3):

Une solution de 3,5-dibromo-4-nitropyrazole (2) (16,0 g, 59 mmoles) dans la DMF (130 ml) a été ajoutée goutte à goutte pendant 20 min à une solution agitée de NaH (2.6 g, 65 mmoles ; dispersion dans l'huile à 60% préalablement lavée à l'hexane sous atmosphère inerte) dans la DMF (80 ml). Après 10 min d'agitation, une solution de (2-Bromo-ethyl)-carbamic acid tert-butyl ester (19.85 g, 88.5 mmol ; obtenu par réaction de 2-bromoethylamine avec di-tert-butyl-dicarbonate) dans la DMF (35 ml) a été additionnée goutte à goutte pendant 10 min. Le mélange réactionnel a été chauffé à 80°C pendant 3 h, puis la DMF a été évaporée sous pression réduite. On rajoute un mélange de DCM / eau (200 ml, 1 /1) au résidu et lave la phase organique à l'eau (100 ml). La phase organique a été séchée sur Na₂SO₄ et le solvant a été évaporé sous pression réduite. Le [2-(3,5-Dibromo-4-nitro-pyrazol-1-yl)-ethyl]-carbamic acid tert-butyl ester (3.6 g, 15%) a été obtenu sous forme d'un solide jaune clair.
1H-RMN (400 MHz, d⁶-DMSO): 7.02 (1 H, t, CH₂CH₂N*H*), 4.29 (2 H, t, C*H*₂CH₂NH), 3.37 (2 H, m, CH₂C*H*₂NH), 1.35 (9 H, s, C*H*₃).

### Synthèse de [2-(5-Benzylamino-3-bromo-4-nitro-pyrazol-1-yl)-ethyl]-carbamic acid tert-butyl ester (4):

Un mélange de. [2-(3,5-Dibromo-4-nitro-pyrazol-1-yl)-ethyl]-carbamic acid tert-butyl ester (3.5 g, 8.45 mmol), EtOH (100 ml) et benzylamine (12,9 g, 117 mmoles) a été chauffé à reflux pendant 1.5 h. Le milieu réactionnel a été concentré au maximum sous pression réduite. Le résidu a été repris dans du DCM (100 ml) et la phase organique a été lavée à l'eau (5 x 50 ml) jusqu'à élimination de tout excès de benzylamine, puis séchée sur Na₂SO₄. Le solvant organique a été évaporé sous pression réduite pour donner le [2-(5-Benzylamino-3-bromo-4-nitro-pyrazol-1-yl)-ethyl]-carbamic acid tert-butyl ester (2.5 g, 67%) sous forme d'un solide jaune clair.
¹H - RMN (400 MHz, d⁶-DMSO): 8.00 (1 H, t, N*H*_{Bn}), 7.32 (5 H, m, *H*_{Bn}), 7.05 (1 H, t, N*H*_{BOC}), 4.70 (2 H, d, *J* = 8 *Hz,* C*H*_{2Bn}), 3.94 (2 H, m, CH₂C*H*₂NH), 3.22 (2 H, m, C*H*₂CH₂NH), 1.35 (9 H, s, C*H*₃).

### Synthèse du bromhydrate de la 2-(2-Amino-ethyl)-2H-pyrazole-3,4-diamine (5):

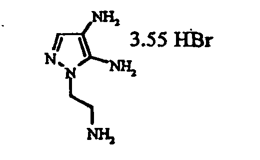

Un mélange de [2-(5-Benzylamino-3-bromo-4-nitro-pyrazol-1-yl)-ethyl]-carbamicacid tert-butyl ester (2.1 g ; 4.76 mmoles) dans EtOH (100 ml) contenant un catalyseur de 5% Pd/C (Type Engelhard, humide à 50%, 0.4 g poids humide) a été hydrogéné dans une autoclave (250 ml) à 15 bars pendant 3 h. Le catalyseur a été filtré sous atmosphère inerte, lavé à l'EtOH et le filtrat a été récupéré dans une solution éthanolique (25 ml) contenant de l'acide bromhydrique (47%, 2 ml). La solution orangée a été évaporée à sec pour donner le 2-(2-Amino-ethyl)-2H-pyrazole-3,4-diamine sous forme de bromohydrate (3.55 HBr) comme solide rose clair (1.5 g, 92%).

| Analyse élémentaire (C₅H₁₁N₄, 3.55 HBr; PM = 428.41 g/mol) | | | | |
|---|---|---|---|---|
| Trouvé: | C : 14.82%, | H: 3.35%, | N : 16.99%, | Br : 62.89% |
| Théorie: | C: 14.02%, | H: 3.42%, | N : 16.35%, | Br : 66.21% |

¹H - RMN (400 MHz, d⁶-DMSO): 9.62 (2 H, S_{large}, *H*Br), 7.98 (2 H, S_{large}, CH₂CH₂N*H*₂), 7.37 (1 H, s, *H*_{pyrazole}), 6.68 (4 H, S_{large}, N*H*₂), 4.17 (2 H, t, C*H*₂CH₂NH₂), 3.21 (2 H, m, CH₂C*H*₂NH₂).

### Exemples de composition tinctoriale en milieu alcalin

On prépare la composition suivante :
- Base diaminopyrazole de formule I 5 x 10⁻³ mole
- Coupleur 5 x 10⁻³ mole
- Alcool oléïque polyglycérolé à 2 moles de glycérol 4,0 g
- Alcool oléïque polyglycérolé à 4 moles de glycérol à 78% de matières actives (M.A.) 5,7 g M.A.
- Acide oléïque 3,0 g
- Amine oléïque à 2 moles d'oxyde d'éthylène vendue sous la dénomination commerciale ETHOMEEN O12 par la société AKZO 7,0 g
- Laurylamino succinamate de diéthylaminopropyle, sel de sodium à 55% de M.A. 3,0 g M.A.
- Alcool oléïque 5,0 g
- Diéthanolamide d'acide oléïque 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse à 35% de M.A. 0,455g M.A.
- Acétate d'ammonium 0,8 g
- Antioxydant, séquestrant q.s.
- Parfum, conservateur q.s.
- Ammoniaque à 20% de NH3 100 g pH = 9,5
   M.A. signifie "matière active"

La base et le coupleur sont tels que définis ci-après.

| **TEINTURES À pH ALCALIN** | | |
|---|---|---|
| **Exemples** | **Base** | **Coupleur** |
| 1 | 2-(2-Amino-ethyl)-2H-pyrazole-3,4-diamine | m-aminophénol |
| 2 | 2-(2-Amino-ethyl)-2H-pyrazole-3,4-diamine | 6-chloro-2-méthyl-5-aminophénol |
| 3 | 2-(2-Amino-ethyl)-2H-pyrazole-3,4-diamine | 2,4-diamino-1-(β-hydroxyéthyloxy) benzène, dichlorhydrate |
| 4 | 2-(2-Amino-ethyl)-2H-pyrazole-3,4-diamine | 2-méthyl-5-amino-phénol |

Au moment de l'emploi, on mélange chaque composition tinctoriale poids pour poids, avec une solution d'eau oxygénée à 20 volumes (6% en poids) dont le pH a été ajusté à environ 2,5 avec de l'acide orthophosphorique.

Le mélange est appliqué sur des cheveux gris à 90% de blancs, naturels, à raison de 5 g pour 0,5 g de cheveux.

Après 30 minutes, les cheveux sont ensuite rincés, lavés avec un shampooing standard, rincés à nouveau et séchés.

La couleur des mèches a été évaluée dans le système L*a*b*, sur cheveux blancs, au moyen d'un spectrophotomètre CM 2002 MINOLTA.

Dans l'espace L*a*b*, la clarté est indiquée par la valeur L* sur une échelle de 0 à 100 alors que les coordonnées chromatiques sont exprimées par a* et b* qui indiquent deux axes de couleur, a* l'axe rouge-vert et b* l'axe jaune-bleu.

Selon ce système, plus la valeur de L est élevée, plus la couleur est claire et peu intense. Inversement, plus la valeur de L est faible, plus la couleur est foncée ou très intense.

| **Exemples** | **Cheveux blancs** | | |
|---|---|---|---|
| | **L*** | **a*** | **b*** |
| Exemple 1 | 35,1 | 27,5 | 13,9 |
| Exemple 2 | 41,9 | 25,0 | 15,2 |
| Exemple 3 | 30,7 | 21,3 | 2,1 |
| Exemple 4 | 41,8 | 27,1 | 24,5 |

Les diaminopyrazoles selon l'invention permettent donc d'obtenir des nuances intenses et chromatiques à pH alcalin.

### Exemple de composition tinctoriale en milieu neutre

On réalise les mêmes formulations que ci-dessus en remplaçant l'ammoniaque par de l'acide citrique en une quantité telle que le pH soit égal à 7.

| **TEINTURE À pH NEUTRE** | | |
|---|---|---|
| **Exemple** | **Base** | **Coupleur** |
| 5 | 2-(2-Amino-ethyl)-2H-pyrazole-3,4-diamine | 2-méthyl-5-aminophénol |

On teint des mèches de cheveux gris à 90% de blancs naturels et permanentés avec la composition tinctoriale 5 et 6 ci-dessus de la même façon que pour la teinture à pH alcalin.

On obtient les nuances suivantes :

| **Exemples** | **Cheveux blancs naturels** | | |
|---|---|---|---|
| | **L*** | **a*** | **b*** |
| Exemple 5 | 41,6 | 17,8 | 17,8 |

A pH neutre, les diaminopyrazoles selon l'invention permettent d'obtenir des nuances intenses.

## Revendications

1. Composé dérivé de diaminopyrazole de formule (I): 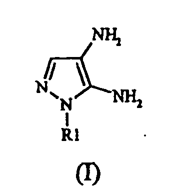 dans laquelle R₁ est un radical linéaire ou ramifié choisi parmi les radicaux aminoalkyles en C₂, C₃, C₄ ou aminoalcényles en C₂, C₃, C₄.

2. Composé de formule (I) selon la revendication 1, tel que le radical aminoalkyle ou aminoalcényle est choisi parmi les radicaux éthyle, n-propyle, iso-propyle, n-butyle, iso-butyle, tertiobutyle, 2-méthylpropyle substitué par 1, 2 ou 3 groupements amino.

3. Composé de formule (I) selon l'une des revendications 1 ou 2 tel qu'il s'agit d'un composé choisi parmi les 2-(2-Amino-ethyl)-2 H-pyrazole-3,4-diamine, 2-(3-Amino-propyl)-2H-pyrazole-3,4-diamine, 2-(2-Amino-propyl)-2H-pyrazole-3,4-diamine, 2-(2,3-Diamino-propyl)-2H-pyrazole-3,4-diamine, 2-(4-Amino-butyl)-2H-pyrazole-3,4-diamine, 2-(3-Amino-butyl)-2H-pyrazole-3,4-diamine, 2-(2-Amino-butyl)-2H-pyrazole-3,4-diamine, 2-(3,4-Diamino-butyl)-2H-pyrazole-3,4-diamine, 2-(2,4-Diamino-butyl)-2H-pyrazole-3,4-diamine, 2-(2,3-Diamino-butyl)-2H-pyrazole-3,4-diamine, 4-(4,5-Diamino-pyrazol-1-yl)-butane-1,2,3-triamine, 2-(3-Amino-1-methylpropyl)-2H-pyrazole-3,4-diamine, 2-(2-Amino-1-methyl-propyl)-2H-pyrazole-3,4-diamine, 2-(1-Aminomethyl-propyl)-2H-pyrazole-3,4-diamine, 2-(3-Amino-1-aminomethyl-propyl)-2H-pyrazole-3,4-diamine, 2-(2-Amino-1-aminomethyl-propyl)-2H-pyrazole-3,4-diamine, 2-(2,3-diamino-1-aminomethyl-propyl)-2H-pyrazole-3,4-diamine, 2-(3-Amino-2-methyl-propyl)-2H-pyrazole-3,4-diamine, 2-(3-Amino-2-aminomethyl-propyl)-2H-pyrazole-3,4-diamine, 2-(3-amino-prop-2-ène)-2H-pyrazole-3,4-diamine, 2-(2-amino-prop-2-ène)-2H-pyrazole-3,4-diamine, 2-(4-amino-but-2-ène)-2H-pyrazole-3,4-diamine, 2-(3-aminobut-3-ène)-2H-pyrazole-3,4-diamine, 2-(1-aminomethyl-prop-2-ène)-2H-pyrazole-3,4-diamine, les ou l'un de leurs sels d'acides physiologiquement acceptables.

4. Composé de formule (I) selon l'une des revendications 1 à 3 tel que les sels physiologiquement acceptables sont des sels d'acides choisis parmi les chlorhydrates, les bromhydrates, les sulfates, les tartrates, les lactates ou les acétates.

5. Composition pour la teinture d'oxydation des fibres kératiniques et en particulier des fibres kératiniques humaines telles que les cheveux, **caractérisée par le fait qu'**elle contient, dans un milieu approprié pour la teinture, comme base d'oxydation au moins un diaminopyrazole de formule (I) selon l'une des revendications 1 à 4.

6. Composition selon la revendication 5, **caractérisée par le fait qu'**elle contient de 0,001 à 10% en poids d'au moins un diaminopyrazole de formule (I) ou de ses sels.

7. Composition selon la revendication 5 ou 6, **caractérisée par le fait que** le milieu approprié pour la teinture (ou support) est constitué par de l'eau ou par un mélange d'eau et d'au moins un solvant organique choisi parmi les alcanols inférieurs en C₁-C₄, les polyols et éthers de polyols, les alcools aromatiques, les produits analogues et leurs mélanges.

8. Composition selon l'une quelconque des revendications 5 à 7, **caractérisée par le fait qu'**elle présente un pH compris entre 3 et 12.

9. Composition selon l'une quelconque des revendications 5 à 8, **caractérisée par le fait qu'**elle contient au moins une base d'oxydation additionnelle choisie parmi les paraphénylènediamines, les bis-phénylalkylènediamines, les para-aminophénols, les ortho-aminophénols, des bases hétérocycliques différentes du diaminopyrazole de formule (I) et leurs sels d'addition avec un acide.

10. Composition selon la revendication 9, **caractérisée par le fait que** la ou les bases d'oxydation additionnelles représentent de 0,0005 à 12% en poids du poids total de la composition tinctoriale.

11. Composition selon l'une quelconque des revendications 5 à 10, **caractérisée par le fait qu'**elle contient au moins un coupleur et/ou au moins un colorant direct.

12. Composition selon la revendication 11, **caractérisée par le fait que** le ou les coupleurs sont choisis parmi les métaphénylènediamines, les méta-aminophénols, les métadiphénols, les dérivés mono- ou polyhydroxylés du naphtalène et les coupleurs hétérocycliques et leurs sels d'addition avec un acide.

13. Composition selon l'une quelconque des revendications 11 ou 12, **caractérisée par le fait que** le ou les coupleurs représentent de 0,0001 à 10% en poids du poids total de la composition tinctoriale.

14. Procédé de teinture des fibres kératiniques et en particulier des fibres kératiniques humaines telles que les cheveux, **caractérisé par le fait que** l'on applique sur ces fibres au moins une composition tinctoriale telle que définie à l'une quelconque des revendications 5 à 13, pendant un temps suffisant pour développer la coloration désirée, soit à l'air, soit à l'aide d'un agent oxydant, éventuellement en présence de catalyseurs d'oxydation.

15. Procédé selon la revendication 14, **caractérisé par le fait que** la coloration est révélée au seul contact de l'oxygène de l'air.

16. Procédé selon la revendication 14 **caractérisée par le fait que** l'on révèle la couleur à pH acide, neutre ou alcalin à l'aide d'un agent oxydant qui est ajouté juste au moment de l'emploi a la composition tinctoriale ou qui est présent dans une composition oxydante appliquée simultanément ou séquentiellement de façon séparée.

17. Procédé selon la revendication 14 ou 16, **caractérisée par le fait que** l'agent oxydant est choisi parmi le peroxyde d'hydrogène, le peroxyde d'urée, les bromates de métaux alcalins, les persels tels que les perborates et persulfates.

18. Dispositif à plusieurs compartiments, ou "kit" de teinture à plusieurs compartiments, dont un premier compartiment renferme une composition tinctoriale telle que définie à l'une quelconque des revendications 5 à 13 et un second compartiment renferme une composition oxydante.

## Claims

1. Compound derived from diaminopyrazole of formula (I): 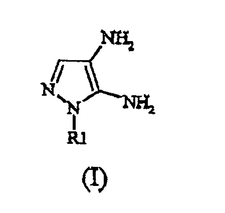 in which R₁ is a linear or branched radical chosen from C₂, C₃ or C₄ aminoalkyl or C₂, C₃ or C₄ aminoalkenyl radicals.

2. Compound of formula (I) according to Claim 1, such that the aminoalkyl or aminoalkenyl radical is chosen from the following radicals: ethyl, n-propyl, isopropyl, n-butyl, isobutyl, tert-butyl or 2-methylpropyl substituted by 1, 2 or 3 amino groups.

3. Compound of formula (I) according to either of Claims 1 and 2, such that it is a compound chosen from 2-(2-amino-ethyl)-2H-pyrazole-3,4-diamine, 2-(3-amino-propyl)-2H-pyrazole-3,4-diamine, 2-(2-amino-propyl)-2H-pyrazole-3,4-diamine, 2-(2,3-diamino-propyl)-2H-pyrazole-3,4-diamine, 2-(4-amino-butyl)-2H-pyrazole-3,4-diamine, 2-(3-aminobutyl)-2H-pyrazole-3,4-diamine, 2-(2-amino-butyl)-2H-pyrazole-3,4-diamine, 2-(3,4-diamino-butyl)-2H-pyrazole-3,4-diamine, 2-(2,4-diamino-butyl)-2H-pyrazole-3,4-diamine, 2-(2,3-diamino-butyl)-2H-pyrazole-3,4-diamine, 4-(4,5-diamino-pyrazol-1-yl)-butane-1,2,3-triamine, 2-(3-amino-1-methylpropyl)-2H-pyrazole-3,4-diamine, 2-(2-amino-1-methyl-propyl)-2H-pyrazole-3,4-diamine, 2-(1-aminomethyl-propyl)-2H-pyrazole-3,4-diamine, 2-(3-amino-1-aminomethyl-propyl)-2H-pyrazole-3,4-diamine, 2-(2-amino-1-aminomethyl-propyl)-2H-pyrazole-3,4-diamine, 2-(2,3-diamino-1-aminomethyl-propyl)-2H-pyrazole-3,4-diamine, 2-(3-amino-2-methyl-propyl)-2H-pyrazole-3,4-diamine, 2-(3-amino-2-aminomethyl-propyl)-2H-pyrazole-3,4-diamine, 2-(3-aminoprop-2-ene)-2H-pyrazole-3,4-diamine, 2-(2-aminoprop-2-ene)-2H-pyrazole-3,4-diamine, 2-(4-aminobut-2-ene)-2H-pyrazole-3,4-diamine, 2-(3-aminobut-3-ene)-2H-pyrazole-3,4-diamine, 2-(1-aminomethylprop-2-ene)-2H-pyrazole-3,4-diamine, or one of the physiologically acceptable acid salts thereof.

4. Compound of formula (I) according to one of Claims 1 to 3, such that the physiologically acceptable salts are acid salts chosen from the hydrochlorides, hydrobromides, sulfates, tartrates, lactates and acetates.

5. Composition for the oxidation dyeing of keratin fibers, and in particular of human keratin fibers such as the hair, **characterized in that** it contains, in a medium that is suitable for dyeing, as oxidation base, at least one diaminopyrazole of formula (I) according to one of Claims 1 to 4.

6. Composition according to Claim 5, **characterized in that** it contains from 0.001% to 10% by weight of at least one diaminopyrazole of formula (I) or salt thereof.

7. Composition according to Claim 5 or 6,
**characterized in that** the medium that is suitable for dyeing (or support) consists of water or of a mixture of water and at least one organic solvent chosen from C₁-C₄ lower alkanols, polyols and polyol ethers, aromatic alcohols, similar products and mixtures thereof.

8. Composition according to any one of Claims 5 to 7, **characterized in that** it has a pH of between 3 and 12.

9. Composition according to any one of Claims 5 to 8, **characterized in that** it contains at least one additional oxidation base chosen from para-phenylenediamines, bis(phenyl)alkylenediamines, para-aminophenols, ortho-aminophenols and heterocyclic bases other than the diaminopyrazole of formula (I), and addition salts thereof with an acid.

10. Composition according to Claim 9, **characterized in that** the additional oxidation base(s) represent(s) from 0.0005% to 12% by weight relative to the total weight of the dye composition.

11. Composition according to any one of Claims 5 to 10, **characterized in that** it contains at least one coupler and/or at least one direct dye.

12. Composition according to Claim 11, **characterized in that** the coupler(s) is (are) chosen from meta-phenylenediamines, meta-aminophenols, meta-diphenols, mono- or polyhydroxylated naphthalene derivatives and heterocyclic couplers, and the addition salts thereof with an acid.

13. Composition according to either of Claims 11 and 12, **characterized in that** the coupler(s) represent(s) from 0.0001% to 10% by weight relative to the total weight of the dye composition.

14. Process for dyeing keratin fibers, and in particular human keratin fibers such as the hair, **characterized in that** at least one dye composition as defined in any one of Claims 5 to 13 is applied to these fibers, for a time that is sufficient to develop the desired coloration, either in air or using an oxidizing agent, optionally in the presence of oxidation catalysts.

15. Process according to Claim 14, **characterized in that** the coloration is revealed solely on contact with atmospheric oxygen.

16. Process according to Claim 14, **characterized in that** the color is revealed at acidic, neutral or alkaline pH with the aid of an oxidizing agent, which is added to the dye composition just at the time of use, or which is present in an oxidizing composition applied simultaneously or sequentially in a separate manner.

17. Process according to Claim 14 or 16, **characterized in that** the oxidizing agent is chosen from hydrogen peroxide, urea peroxide, alkali metal bromates and persalts such as perborates and persulfates.

18. Multi-compartment device or multi-compartment dyeing "kit", a first compartment of which contains a dye composition as defined in any one of Claims 5 to 13, and a second compartment of which contains an oxidizing composition.

## Patentansprüche

1. Diaminopyrazolderivat nach Formel (I): 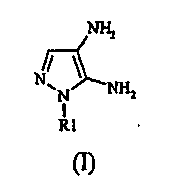 wobei R₁ für ein geradkettiges oder verzweigtes Radikal steht, welches aus C₂-, C₃-, C₄-Aminoalkylradikalen oder C₂-, C₃-, C₄-Aminoalkenylradikalen gewählt wird.

2. Verbindung nach Formel (I) nach dem Anspruch 1, wobei das Aminoalkyl- oder Aminoalkenylradikal aus Ethyl-, n-Propyl-, iso-Propyl-, n-Butyl-, iso-Butyl-, tert.-Butyl-, 2- Methylpropylradikalen gewählt ist und wobei als Substituenten 1, 2 oder 3 Aminogruppen vorhanden sind.

3. Verbindung nach Formel (I) nach einem der Ansprüche 1 oder 2, wobei es sich um eine Verbindung handelt, die aus 2-(2-Aminoethyl)-2H-pyrazol-3,4-diamin, 2-(3-Aminopropyl)-2H-pyrazol-3,4-diamin, 2-(2-Aminopropyl)-2H-pyrazol-3,4-diamin, 2-(2,3-Diaminopropyl)-2H-pyrazol-3,4-diamin, 2-(4-Aminobutyl)-2H-pyrazol-3,4-diamin, 2-(3-Aminobutyl)-2H-pyrazol-3,4-diamin, 2-(2-Aminobutyl)-2H-pyrazol-3,4-diamin, 2-(3,4-Diaminobutyl)-2H-pyrazol-3,4-diamin, 2-(2,4-Diaminobutyl)-2H-pyrazol-3,4-diamin, 2-(2,3-Diaminobutyl)-2H-pyrazol-3,4-diamin, 4-(4,5-Diaminopyrazol-1-yl)-butan-1,2,3-triamin, 2-(3-Amino-1-methylpropyl)-2H-pyrazol-3,4-diamin, 2-(2-Amino-1-methylpropyl)-2H-pyrazol-3,4-diamin, 2-(1-Aminomethylpropyl)-2H-pyrazol-3,4-diamin, 2-(3-Amino-1-aminomethylpropyl)-2H-pyrazol-3,4-diamin, 2-(2-Amino-1-aminomethylpropyl)-2H-pyrazol-3,4-diamin, 2-(2,3-Diamino-1-aminomethylpropyl)-2H-pyrazol-3,4-diamin, 2-(3-Amino-2-methylpropyl)-2H-pyrazol-3,4-diamin, 2-(3-Amino-2-aminomethylpropyl)-2H-pyrazol-3,4-diamin, 2-(3-Aminoprop-2-en)-2H-pyrazol-3,4-diamin, 2-(2-Aminoprop-2-en)-2H-pyrazol-3,4-diamin, 2-(4-Aminobut-2-en)-2H-pyrazol-3,4-diamin, 2-(3-Aminobut-3-en)-2H-pyrazol-3,4-diamin, 2-(1-Aminomethylprop-2-en)-2H-pyrazol-3,4-diamin sowie aus deren oder einem ihrer physiologisch unbedenklichen Salze gewählt wird.

4. Verbindung nach Formel (I) nach einem der Ansprüche 1 bis 3, wobei es sich bei den physiologisch unbedenklichen Salzen um Salze von Säuren handelt und diese Salze aus den Chlorhydraten, den Bromhydraten, des Sulfaten, den Tartraten, den Lactaten oder den Acetaten gewählt sind.

5. Zusammensetzung für das Färben durch Oxidation von Keratinfasern und insbesondere von menschlichen Keratinfasern wie etwa Haaren, **dadurch gekennzeichnet, dass** sie in einem Milieu, welches zum Färben geeignet ist, mindestens ein Diaminopyrazol nach Formel (I) nach einem der Ansprüche 1 bis 4 als Oxidationsbase enthält.

6. Zusammensetzung nach dem Anspruch 5, **dadurch gekennzeichnet, dass** sie 0,001 bis 10 Gewichts% mindestens eines Diaminopyrazols nach Formel (I) oder an dessen Salzen enthält.

7. Zusammensetzung nach den Ansprüchen 5 oder 6, **dadurch gekennzeichnet, dass** das Milieu (oder die Trägersubstanz), welche(s) zum Färben geeignet ist, aus Wasser besteht, oder aber aus einer Mischung von Wasser mit mindestens einem organischen Lösemittel, das aus den kurzkettigen C₁-C₄-Alkanolen, den Polyolen und Polyolethern, den aromatischen Alkoholen sowie analogen Substanzen und Mischungen daraus gewählt ist.

8. Zusammensetzung nach einem beliebigen der Ansprüche 5 bis 7, **dadurch gekennzeichnet, dass** sie einen pH-Wert zwischen 3 und 12 aufweist.

9. Zusammensetzung nach einem beliebigen der Ansprüche 5 bis 8, **dadurch gekennzeichnet, dass** sie mindestens eine zusätzliche Oxidationsbase enthält, die aus den para-Phenylendiaminen, den bis-Phenylalkylendiaminen, den para-Aminophenolen, den ortho-Aminophenolen, den heterozyklischen Basen, die nicht dem Diaminopyrazol nach Formel (I) entsprechen, sowie deren Additionssalzen mit einer Säure gewählt ist.

10. Zusammensetzung nach dem Anspruch 9, **dadurch gekennzeichnet, dass** die zusätzliche(n) Oxidationsbase(n) zwischen 0,0005 und 12 Gewichts% des Gesamtgewichts der Färbezusammensetzung ausmacht/ausmachen.

11. Zusammensetzung nach einem beliebigen der Ansprüche 5 bis 10, **dadurch gekennzeichnet, dass** sie mindestens einen Kuppler und/oder mindestens einen Direktfarbstoff enthält.

12. Zusammensetzung nach dem Anspruch 11, **dadurch gekennzeichnet, dass** der oder die Kuppler aus den meta-Phenylendiaminen, den meta-Aminophenolen, den meta-Diphenolen, den mono- oder polyhydroxylierten Derivaten des Naphthalins und den heterozyklischen Kupplern sowie deren Additionssalzen mit einer Säure gewählt ist/sind.

13. Zusammensetzung nach einem beliebigen der Ansprüche 11 oder 12, **dadurch gekennzeichnet, dass** der oder die Kuppler zwischen 0,0001 und 10 Gewichts% des Gesamtgewichts der Färbezusammensetzung ausmacht/ausmachen.

14. Verfahren zum Färben von Keratinfasern und insbesondere von menschlichen Keratinfasern wie etwa Haaren, **dadurch gekennzeichnet, dass** mindestens eine Färbezusammensetzung nach der Begriffsbestimmung in einem beliebigen der Ansprüche 5 bis 13 für eine Zeitdauer auf die Fasern angewendet wird, die ausreicht, um die gewünschte Färbung zu entwickeln, und zwar entweder an der Luft oder mit Hilfe eines Oxidationsmittels, sowie möglicherweise in Gegenwart von Oxidationskatalysatoren.

15. Verfahren nach dem Anspruch 14, **dadurch gekennzeichnet, dass** der Kontakt mit nur Luftsauerstoff ausreicht, um die Färbung zu entwickeln.

16. Verfahren nach dem Anspruch 14, **dadurch gekennzeichnet, dass** die Farbe bei saurem, neutralem oder alkalischem pH-Wert mit Hilfe eines Oxidationsmittels entwickelt wird, welches erst zum Zeitpunkt der Anwendung der Färbezusammensetzung zugesetzt wird oder welches in einer oxidierenden Zusammensetzung enthalten ist, die separat angewendet wird, und zwar zum gleichen Zeitpunkt oder nachfolgend.

17. Verfahren nach den Ansprüchen 14 oder 16, **dadurch gekennzeichnet, dass** das Oxidationsmittel aus Wasserstoffperoxid, Harnstoffperoxid, Erdalkalimetallbromaten und Per-Salzen wie etwa Perboraten und Persulfaten gewählt ist.

18. Vorrichtung aus mehreren Teilbehältnissen oder Färbe-"Kit" mit mehreren Teilbehältnissen, von denen ein erstes Teilbehältnis eine Färbezusammensetzung nach einem beliebigen der Ansprüche 5 bis 13 enthält und von denen ein zweites Teilbehältnis eine Oxidationsmittelzusammensetzung enthält.
